(19) Europäisches Patentamt
European Patent Office
Office européen des brevets

(11) **EP 2 305 158 A1**

(12) **EUROPEAN PATENT APPLICATION**
published in accordance with Art. 153(4) EPC

(43) Date of publication:
06.04.2011 Bulletin 2011/14

(51) Int Cl.:
*A61B 18/02* (2006.01)   *A61F 7/10* (2006.01)

(21) Application number: 09797879.5

(22) Date of filing: 10.07.2009

(86) International application number:
PCT/JP2009/062629

(87) International publication number:
WO 2010/007954 (21.01.2010 Gazette 2010/03)

(84) Designated Contracting States:
AT BE BG CH CY CZ DE DK EE ES FI FR GB GR HR HU IE IS IT LI LT LU LV MC MK MT NL NO PL PT RO SE SI SK SM TR
Designated Extension States:
AL BA RS

(30) Priority: 15.07.2008   JP 2008184264
10.10.2008   JP 2008263892

(71) Applicant: **DGS Computer**
**Hachiouji-shi**
**Tokyo 192-0364 (JP)**

(72) Inventors:
• **IWATA Kansei**
**Hachiouji-shi**
**Tokyo 192-0363 (JP)**
• **IWATA Yasushi**
**Hachouji-shi**
**Tokyo 192-0363 (JP)**

• **NAGASAWA Taisuke**
**Machida-shi**
**Tokyo 194-0212 (JP)**
• **KAWAMURA Masafumi**
**Tokyo 161-0032 (JP)**
• **IZUMI Yotaro**
**Tokyo 158-0095 (JP)**
• **NAKATSUKA Seishi**
**Tokyo 157-0073 (JP)**
• **INOUE Masanori**
**Tokyo 155-0033 (JP)**
• **TSUKADA Norimasa**
**Tokyo 166-0003 (JP)**
• **YASHIRO Hideki**
**Hiratsuka-shi**
**Kanagawa 254-0042 (JP)**

(74) Representative: **Wagner, Karl H.**
**Wagner & Geyer Partnerschaft**
**Patent- und Rechtsanwälte**
**Gewürzmühlstrasse 5**
**80538 München (DE)**

(54) **CRYOTHERAPY PLANNING DEVICE AND CRYOTHERAPY DEVICE**

(57)    The present invention relates to a treatment device utilized in the freezing treatment method and its treatment planning device, and has an object to settle a freezing period defrosting period according to a size of a treatment portion.

A cryotherapy device comprises a gas supply-exhaust system 100, a control system 200 therfore and a freezing probe system 300. The gas supply-exhaust system 100 supplies a freezing gas and a defrosting gas to a probe 60 of the freezing probe system 300 to freeze and defrost the treatment portion surrounding the tip of the probe 60 by the Joule Thomson effect. The control system 200 controls the gas supply-exhasust system 100 and makes treatment planning data for this control. The treatment planning data includes a freezing · defrosting sequence to determine the freezing period and the defrosting period. The determination of this sequence is performed by the computer in the control system 200. Further, this sequence is determined corresponding to the focus treatment size according to the freezing defrosing characteristics of the tissue.

[Figure 1]

## Description

### Field of the Invention

This invention relates to, for performing the extreamly low temperature therapy, the cryotherapy planning device, and the cryotherapy device utilizing it.

### Background of the Invention

In recent years, the attention is paid to the cryotherapy which treats a patient's affected portion using extreamly low temperature.
This cryotherapy is a treatment that, in the state that a double pipe (coaxial needle) whose tip is acute is applied to the patient's body surface, a long thin introducer is inserted along the central axis of the double pipe and stabbs the affected portion; the double pipe is advanced to the affected portipon along the introducer and penetrates the affected portion; the coaxial needle may be made to penetrate directly; then, the introducer is extracted, and instead, a freezing terminal (probe) is inserted and loaded along the hollow shaft in the double pipe; a freezing gas (gas and liquid both can be used) and a defrosting gas (gas and liquid both can be used) are supplied; and the affected portion is made necrosis by repeating freezing and defrosting (fusion) the affected portion for a short time.
Applicant filed various patent applications concerning the cryotherapy, and have already been open to public.

Patent document 1: JP, 2007-167100, A
Patent document 2: JP, 2007-167101, A
Patent document 3: JP, 2007-295953, A

### Description of the Invention

### Problem to be solved by the Invention

Conventional freezing therapeutic devices are still not practical technically. For example, a freezing temperature and its freezing time width, and a defrosting temperature and its defrosting time width are settled experimantally. Hereafter, the development of practical and reliable devices having a high safety will be desired.
It is an object of the present invention to provide a cryotherapy planning device and a cryotherapy device which enable a proper freezing therapy based on an analysis result of a freeaing · defrosting mechanism in a focus organization.
It is a concrete object of the present invention to provide a cryotherapy plannning device and a cryotherapy device wherein a freezing temperature · freezing time width and a defrosting temperature · defrosting time width obtained quantitatively by clarifying and functionizing the characteristic of freezing defrosting in the focus organization can be utilized for the control of the freezing gas and the defrosting gas.
It is another object of the present invention to provide a cryotherapy planning device and a cryotherapy device which enable safely to purge a freezing gas in an evapolation freezing method utilizing the extreamly low temperature liquefied gas as the freezing gas.

### Means for Solving the Problem

The present invention provides a cryotherapy planning device comprising, in a cryotherapy device wherein a freezing and a defrosting can be performed in a freezing period which freezes a treatment portion and a defrosting period in which its defrosting is performed, by a freezing probe having a predetermined section size, that said freezing period and said defrosting period are settled according to an organization and a focus treatment size of the treatment portion.
The present invention provides, further, a cryotherapy planning device comprising, in a cryotherapy device wherein a freezing and a defrosting can be performed in a freezing period which freezes a treatment portion and a defrosting period in which its defrosting is performed, that said freezing period and said defrosting period are settled according to a section size of a freezing probe, and an organization and a focus treatment size of the treatment portion.
The present invention provides, further, a cryotherapy planning device wherein a relation between a kind of diameter of a freezing probe and a focus treatment size, obtained by a diameter size of the freezing probe and a freezing limit size settled according to the diameter size, is memorized as data, and, by using this data, a freezing probe to be used and a focus treatment size are determined.
The present invention provides, further, a cryotherapy planning device wherein a relation between a diameter size of a freezing probe, a freezing limit size settled according to the diameter size and a time width approaching to the freezing limit size, is memorized as data, and, by using this data, a freezing probe to be used, a focus treatment size and a freezing time are determined.

The present invention provides, further, a cryotherapy planning device wherein a relation between a diameter size of a freezing probe, a freezing limit size settled according to the diameter size and a time width approaching to the freezing limit size, is memorized as data, and, by using this data, a freezing probe to be used, a focus treatment size, a freezing time, a defrosting period and/or a repetition of cycle of its freezing and defrosting are determined.

The present invention provides, further, a cryotherapy planning device wherein above relation is controlled by a following equation settled by a diameter of a freezing probe, when y is a freezing size and c is a freezing limit size,

$y = a \exp(bt) + C$ wherein exp is an exponential function, a, b and c are coefficients settled by a treatment portion and a diameter of a probe, being in a relation of $a < 0$, $b < 0$, $c > 0$, and c is the freezing limit size.

The present invention provides, further, a cryotherapy device comprising a freezing probe, a gas control means to send a freezing gas for freezing a treatment portion during a freezing period and to send a defrosting gas for its defrosting during a defrosting period, to the freezing probe, means for setting a freezing probe to be used, a focus treatment size, a freezing time T1 and a defrosting time T2 by utilizing memorized data of a relation between the diameter size of the freezing probe, a freezing limit size settled according to the diameter size and a time width approaching to the freezing limit size, and commanding means for sending a control command to said gas control means so as to freeze at the settled freezing time T1 and to defrost at defrosting time T2.

The present invention provides, further, a cryotherapy device wherein the above relation is controlled by a following equation settled by a diameter of a freezing probe, when y is a freezing size and c is a freezing limit size,

$y = a \exp(bt) + c$

wherein exp is an exponential function, a, b and c are coefficients settled by a treatment portion and a diameter of a probe, being in a relation of $a < 0$, $b < 0$, $c > 0$, and c is the freezing limit size.

The present invention provides, further, in a freezing treatment period planning device which obtains a freezing period $T_1$ for freezing a treatment portion and a defrosting period $T_2$ to defrost it by utilizing a freezing probe, a freezing treatment period planning device wherein the freezing period $T_1$ for freezing the treatment portion surrounding the freezing probe by a freezing gas is settled based on a time t obtained by solving an equation of

$y - A \ln(t) + B$ (ln is natural logarithm)

wherein A and B are constants settled by an organization of the treatment portion and y is a focus treatment size and the defrosting period $T_2$ is settled based on a time t obtained by solving an equation of

$Z = C - D \ln(t)$

wherein Z is a defrosting size, and C and D are constants settled by an organization of the treatment portion.

The present invention provides, further, a freezing treatment period planning device wherein, when a freezing period and a defrosting period are made as 1 cycle, the number of a repetition of the cycle is settled.

The present invention provides, further, a freezing treatment period planning device wherein a purge period for purging a freezing gas is added to the freezing period.

The present invention provides, further, a cryotherapy device comprising a freezing probe, a gas control means to send a freezing gas for freezing a treatment portion during a freezing period and to send a defrosting gas for its defrosting during a defrosting period, to the freezing probe, means for setting a freezing period $T_1$ which is settled based on a time t obtained by solving an equation of $y - A l_n(t) + B$ ($l_n$ is natural logarithm) wherein A and B are constants settled by an organization of the treatment portion and y is a focus treatment size, and a defrosting period $T_2$ which is settled based on a time t obtained by solving an equation of $Z = C - D l_n(t)$ ($l_n$ is natural logarithm)

wherein Z is a defrosting size, and C and D are constants settled by an organization of the treatment portion, and commanding means for sending a control command to said gas control means so as to freeze for the settled freezing period $T_1$ and to defrost for the defrosting period $T_2$.

The present invention provides, further, a cryotherapy device wherein said setting means settle the number of a repetition of cycle, when a freezing period and a defrosting period are made as 1 cycle, and said commanding means send control commands for freezing and defrosting to said gas control means according to the cycle.

The present invention provides, further, a cryotherapy device wherein said setting means add a purge period for purging a freezing gas in case that a liquid freezing gas is utilized to said freezing period, and said commanding means send control commands for freezing, defrosting and purging according to these periods.

Further, the present invention provides a cryotherapy device wherein a gas of a vaporized gas generated in a liquid freezing gas source is used as the purge gas in the purge period.

## Effects of the Invention

According to this invention, since a freezing period and a defrosting period can be determined based on the organization of the treatment portion and the focus treatment size, a proper time and a proper treatment can be realized.

**Best Mode Carrying Out the Invention**

A freezing and a defrosting depend on making to the low temperature and making to the high temperature. There are examples for the freezing and the defrosting by using a Joule Thomson effect and by evaporating a low temperature liquefied gas.

The Joule · Thomson effect is a thermal phenomenon occurred when a predetermined pressure of a gas having a constant temperature, such as room temperature, is suddenly lowered. There are examples to become lower temperature and higher temperature, according to a kind of gas. These are utilized for the freezing and the defrosting. These are realized that, for example, when a probe has a structure so that the gas expands suddenly at the tip thereof, and ordinaly temperature argon gas (Ar gas) and helium (He gas) of high pressure of 30MP are led to the tip of the probe. Freezing temperature of - 125°C is obtained in Ar gas, and the defrosting temperature of +20°C is acquired in He gas.

A low temperature liquefied gas is used in evaporation of a liquefied gas. For example, in nitrogen ($N_2$) gas, it liquefies by cooling with the high pressure of 70MP, and this liquid temperature is about -195°C. On this evaporation, many quantity of heat is taken from the circumference, and makes it freeze. The defrosting is realized by sending out a high-temperature fusion gas (or liquid).

The definition of a focus treatment portion and a focus treatment size is clarified, here.

A part which is treated by one freezing probe in the cycle of one time or multiple times will be called a focus treatment portion, and the size of this focus treatment portion will be called a focus treatment size. The focus treatment portion is as follows.

(1) An example of a focus of small spot size or that they are generated dispersively. In this case, the focus of spot size itself is the focus treatment portion. A size of each focus is the focus treatment size.
(2) A first example of a focus having a big volume or area size. In this case, when the treatment is performed by utilizing a probe of large-diameter size, the whole focus is the focus treatment portion and its size is the focus treatment size.
(3) A second example of a focus having a big volume or area size. In this case, when the focus is segmented continuously and each segment is treated by a probe of small-diameter size, each segment is the focus treatment portion and its size is the focus treatment size.

The inventor of this application has discovered the relation between a freezing temperature and its freezing size. It is described below.

When freezing a certain focus treatment portion, a freezing is performed over a certain time with a cetain freezing temperature. However, if a freezing temperature is settled, the maximum freezing size is settled with this freezing temperature, and the freezing size does not expand beyond it even though the freezing time is extended. And in the intermediate freezing time until it reaches the maximum freezing size, the freezing size is settled with a certain functional relation to the freezing time.

This is applied to the spot freezing source. The spot freezing source is a freezing source of a spot size, and, in this invention, the tip of the freezing probe serves as this spot freezing source. If the diameter size of the spot freezing source is as extremely small as being disregarded, the freezing size $\delta$ becomes

$$[\text{Numerical Formula 1}] \qquad \delta \leqq \delta_{\max}.$$

Here, the freezing size $\delta$ is settled according to the length of the freezing time.
When the diameter size ro of the freezing probe is taken into consideration, it becomes

$$[\text{Numerical Formula 2}] \qquad \delta - r_0 \leqq \delta_{\max}.$$

The relation between the focus treatment size to be treated and the freezing temperature of the freezing probe is that the focus treatment size needs to be equal or smaller than the maximum freezing size $\delta_{\max}$ settled by its freezing temperature. This maximum freezing size $\delta_{\max}$ is the constant c which is settled by the numerical formula 4 described later.

The defrosting is the reverse of the freezing and, fundamentally, the same view as the freezing can be applied.

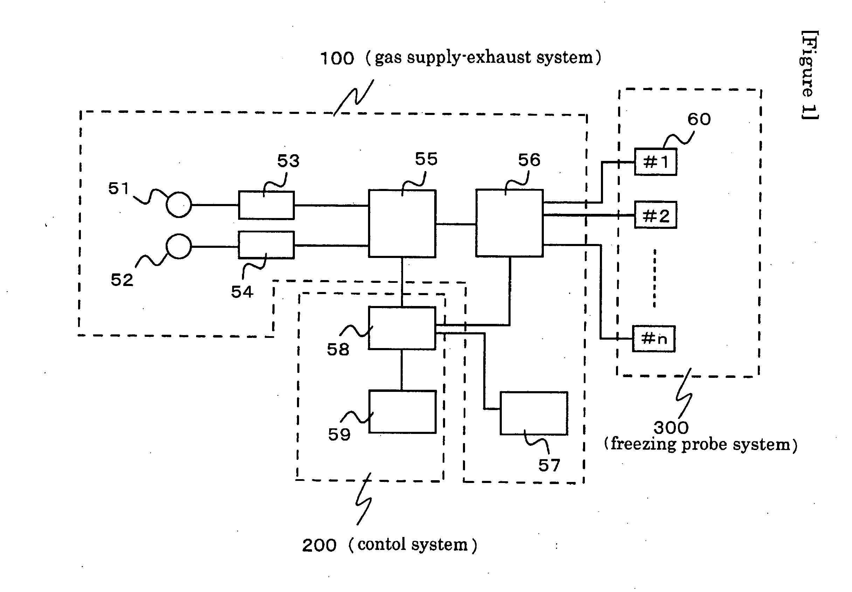

Figure 1 is a general viw of an embodiment of a cryotherapy device of the present invention using the Joule · Thomson effect. This treatment device comprises a gas supply-exhaust system 100, a control system 200 and a freezing probe system 300.

The gas supply-exhaust system 100 comprises a source 51 of gas (for example argon gas) of a room temperature with a high pressure, a source 52 of gas (for example hellium gas) of a room temperature with a high pressure, gas stabilizers 53 and 54, a gas switching pressure gauge portion 55, a distribution changeover portion 56 and a gas exhaust control portion 57.

The source 51 of high pressure gas functions as a gas source for freezing by making extremely low temperature (in argon, about -125°C) based on the Joule Thomson effect, and the source 52 of high pressure gas functions for defrosing from the freezing state by making high temperature (in hellium, about +25°C) based on the Joule Thomson effect.

The gas stabilizers 53 and 54 are for making the pressure of the high pressure gas from the gas source 51 and 52 constant.

The gas switching pressure gauge portion 55 is the portion containing a switch for changing a passage of gases from the gas sources 51 and 52, an electromagnetic valve and a branching pipe as the switching means, and a variou kinds of monitoring instruments.

The distribution changeover portion 56 is the distributing and changing means for selecting a probe to be used from the probes 60 having a plurality of probes and for selecting gas supplied to it.

The gas exhaust control portion 57 is the means for exhausting the used gas from the probes 60, and includes the purging means in a treatment device utilizing the evaporation phenomenon wherein the liquid gas is used for freezing, described later.

The control system 200 comprises the control · measurement portion 58 and the control computer 59.

The control computer 59 has a various kinds of data and the treatment programs, and performs the instruction and the monitor of the treatment execution. The treatment execution is attained by controlling the gas switching and pressure gauge portion 55, the gas distribution · changeover portion 56 and the exhaust portion 57. The control computer 59 makes control commands required for those controls, and sends to the control · measurement portion 58.

The control · measurement portion 58 generates a control signal to each portion 55, 56 and 57 according to the control command from the control computer 59, and controls the specific treatment means. The control · measurement portion 58, further, takes measured signals being data and status data of various instruments in each portion 55, 56 and 58, and monitors the control and the operation. For example, the monitoring data is transmitted through the distribution and changeover portion from sensors, such as a thermo couple equipped in the probe, and is taken into the computer 59.

In addition, the control computer 59 has variou data containing the patient's ID information, the treatment history, the name of a desease, the treatment portion and its position including the name of an organ, the focus size, physiological data including the blood pressure and the blood sugar level, etc., and the photographic image data of the affected portion, etc.

The treatment program is a software including the execution process of cryotherapy and is formed with the treatment planning and said various data.

The freezing system 300 has plural probes (#1 ~ #n) 60. The probes 60 are the same form or, also the different form each other, and there are examples of using one and of using plural (2 or 3 pieces) concurrently. These numbers and the usages are included in the treatment program. The size of the diameter size, the length of the probe, the quantity of the gas, and various contents are called the form here. The wall of the probe is equipped with the thermo couple, and the temperature of the probe is sent to the control-measurement portion.

The treatment program has the contents of processing containing the treatment procedure, the freezing defrosting sequence (the number n of the cycle, when the freezing and defrosing are made as 1 cycle, the time width T of the 1 cycle), the risk management of the treatment, the monitor management of the various kinds of living body watching equipments and devices including X-ray CT device, the electrocardio device and the manometer, etc for monitoring the treatment execution and watching.

The negative pressure system about the exhaust is explained.

High pressure gas is sent through the distribution and changeover mechanism to the probe 60 in the probe system 300, expands in the probe according to the Joule-Thomson effct, and is discharged through the exhaust system in a negative pressure. In this exhaust system, the negative pressure is held by the negative pressure generating mechanism installed in the exhaust control system 57, and prompt discharge is carried out. A negative pressure monitoring sensor is equipped in the negative pressure generating mechanism, and an output of this sensor is sent to the computer 59. The computer 59 watches the negative pressure. If negative pressure turns it to positive, the probe will be filled with a sending-out high pressure gas, and will result in a very dangerous state. When it is judged that this negative pressure changed to positive pressure or high positive pressure, the computer 59 urgently stops the control system.

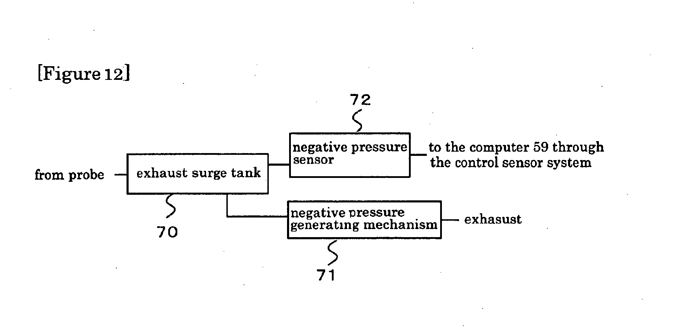

Such embodiment is shown in Figure 12. This exhausting system has an exhaust surge tank 70, a negative pressure generating mechanism 71 and a negative pressure sensor 72. The exhaust gas from an exhaust path of the probe goes into the exhaust surge tank 70. The exhaust surge tank 70 is maintained at a negative pressure by the negative pressure generating mechanism 71. The negative pressure sensor 72 detects that a return exhaust system of the probe is kept in a negative pressure and is in an aspiration state. The output of the sensor is always inputted into the computer 59 and watched that it is normal. When it changes into the contrary state, the control system 57 is immediately stopped and the sending-out of the high pressured gas is stopped.

In the treatment program, the freezing · defrosting sequence is especially concerned with this present invention. In the freezing · defrosting sequence, the number n of the cycle is usually a value of more than 1, and can be changed variously, such as n = 2 or n = 3. The time width T of the cycle can also be changed variously. The time width T of 1 cycle is the total value of the freezing time width (freezing period) $T_1$ and the defrosting time width (defrosting period) $T_2$. The width $T_2$ corresponds to the time width necessary for defrosting the freezing in the width $T_1$.

The number n of cyle and the time width T of 1 cycle are decided with the treetment size (diameter, volume or cross sectional area) of the focus of the freezing object. When the focus treatment size is large, at least either one of n and/or T is settled large. For example, in case n = 3, it is adjusted by T; when T is fixed, n is made large or small according to the size. There is also an example wherein both of n and T are changed.

Hereinafter, the many features, functions and effects of the present invention are explained, in the treatment program execution, by limiting to the execution of freezing defrosting sequence. In Figure 1, it is also shown that only the composition along with such meaning.

First, it is described that the determination method of 1 cycle time width T according to the dimension of the focus treatment size of the freezing treatment portion in case that the number of cycle n is n = 3.

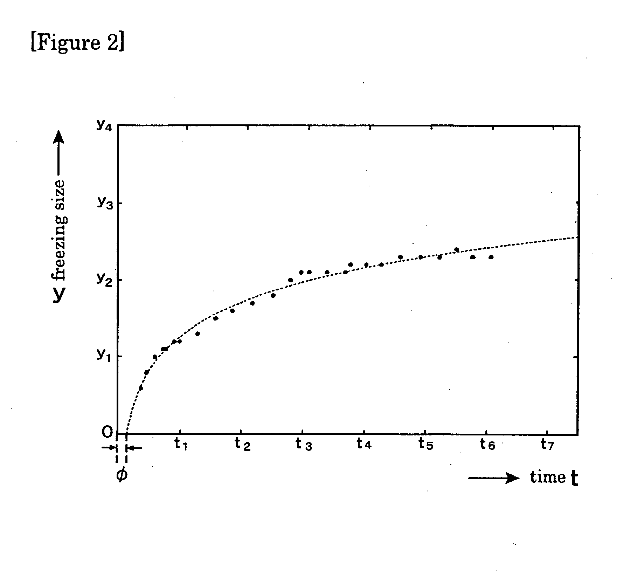

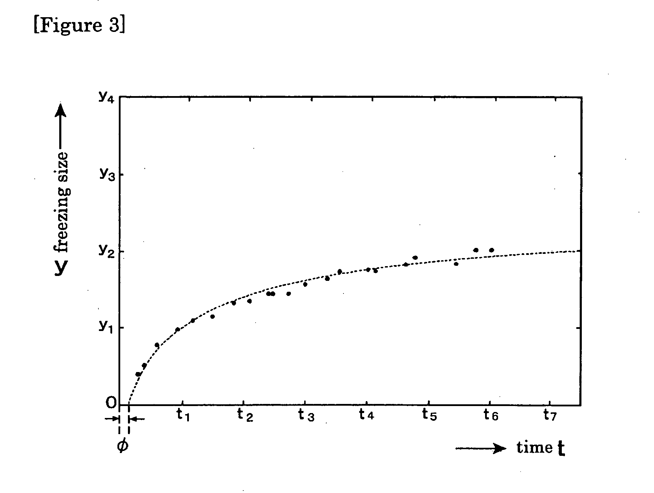

When the focus treatment size is large, the large freezing energy is required. This freezing energy depends on the length of 1 cycle time T. The applicant of this application searched for the relation between the focus treatment size and the freezing time by the animal experiment under the use of freezing probe whose section size (a diameter size in a circle, and an area size is also included) is fixed. Figure 2 shows an example of freezing in a lung, and Figure 3 shows an example of freezing in a liver. The transverse designates the freezing time t and the vertical axis designates the freezing size (diameter) y generated by the freezing. The freezing is also called the congelation. Because the freezing size y corresponds to the freezing temperature AT (extremely low temperature, such as around -125°C), the vertical axis may be designated according to the scale of the freezing temperature AT. For example, the freezing size y is values from several millimeters to several ten millimeters, and the freezing time t is values of from several ten seconds to several hundreds of seconds.

The example of approximation functions of plotted points in Figures 2 and 3 becomes, in the method of least squeres, a following formula.

$$[\text{Numerical Formula 3}] \qquad y = A \ln (t) + B.$$

Here, it is designated that A and B are values mostly settled with the kind (organization), the state and the size of a region of organ, such as a lung and a liver, the freezing temperature depending on the freezing gas to be used, etc., and the diameter of the probe, and In is a natural logarithm. The time $\Phi$ (= exp (-B/A)) at the start of the function in Figures 2 and 3 corresponds to the diameter of the probe.

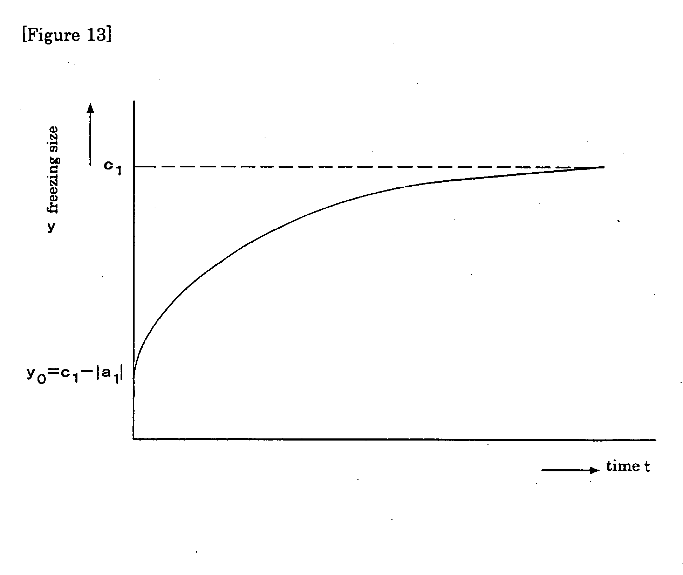

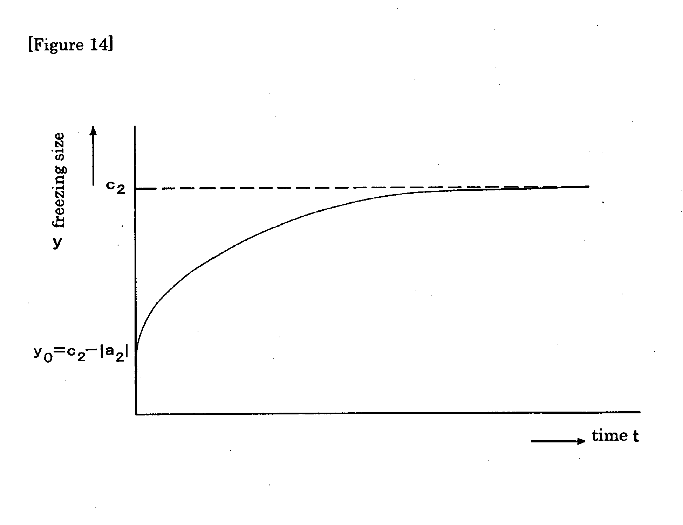

Figures 13 and 14 are the examples expressed Figures 2 and 3 on another scale. However, the experimental data (dots) are omitted in Figures 13 and 14. Figures which shifted Figures 2 and 3 to left and enlarged time enough are Figures 13 and 14. The intersections y = $y_0$ ($c_1$ — $|a_1|$) and ($c_2$ - $|a_2|$) of y-axes at the transverses t = 0 show the diameters of the probes. That is, the diameter yo of the probe was made into the initial value of the freezing. To enlarge time enough means here to have taken the time beyond the time for the freezing limit which is that the freezing size cannot advance anymore.

The example of approximation functions based on the method of least squeres of Figures 13 and 14 becomes a following formula.

$$[\text{Numerical Formula 4}] \qquad y = a \exp (bt) + c.$$

Here, it is designated that a, b and c are values mostly settled with the kind (organization), the state and the size of a regon of organ, such as a lung and a liver, the freezing temperature depending on the freezing gas to be used, etc., and the diameter of the probe, and are a < 0, b < 0 and c > 0.

That is, coefficients A, B, a, b, and c are considered to be uniquely decided according to the size of the diameter of the probe when the kind and the state of the focus treatment and the freezing temperature are settled.

Differences between the numerical formula 3 and the numerical formula 4 are as follows;

(1) The numerical formula 4 is a formula in consideration of the freezing limit size. The freezing limit size is the maximum size of the freezing area generated, when the probe stabbes the focus, around the stabbed portion. When a focus organ is specified, the freezing limit size is decided by the diameter of the probe. The larger the diameter becomes, the larger its size becomes; and the smaller the diameter, the smaller its size.

The value of y becomes $y = c$ at $t = \infty$ in the numerical formula 4. This value c is the freezing limit size. In practice, y mostly saturates in a limited and short time width, such as 3 or 7 minuites, instead of $t = \infty$, and becomes to the freezing limit size c ($c_1$ in Figure 13 and $c_2$ in Figure 14). Therefore, the freezing time width for a long time is unnecessary. In the numerical formula 4, the value $c + a$ of y at $t = 0$ shows the diameter of the probe. Since $a < 0$, $c - |a|$ becomes the diameter of the probe.

(2) In the numerical formula 3, $y = \infty$ at $t = \infty$, on the expression, and is not saturated. Therefore, it is not employable for the freezing time of the time width which is saturated. On the contrary, it is used for determining the freezing time wherein the freezing is not saturated.

Next, each meaning and utilization of the numerical formula 3 and the numerical formula 4 is explained.

(1) There is a meaning that the numerical formula 3 is applicable to the determination of the freezing time for the freezing size which does not reach to the freezing limit size. The example of use is described later.

(2) There is a meaning that the numerical formula 4 is applicable to carry out a freezing of the size near to the freezing limit size. Specifically, it becomes a view as follows.

i. There is a meaning that a proper treatment is enabled. Since the treatment size is made to correspond to the freezing limit size, the proper treatment of only the focus, without damaging the normal organization arround the treatment focus, can be attained. Further, since the freezing limit size is obtained by saturation in 3 or 5 minutes, the freezing time for a long time is unnecessary and the rapid treatment can be attained.

ii. There is meaning that the freezing probe having a proper diameter can be chosen. The freezing limit size is basically determined by the diameter of the freezing probe. Therefore, when the treatment size is settled, the freezing probe having the diameter corresponding to it can be chosen, and more suitable treatment can be taken. For example, the diameter sizes of the probe are various, such as 1mm, 2mm and 3mm. When the freezing limit size of each diameter size described above is $c_1$, $c_2$ and $c_3$, the probe of 1mm is chosen for the focus treatment size of $c_1$, and the probe of 2mm is chosen for $c_2$. There may be the case that not accord correctly. If it is middle size of $c_1$ and $c_2$, for example, 1mm sized can be used in 2 steps overlapping a partial area.

iii. There is an example that the freezing size is made below the freezing limit size.

At that time, the freezing limit size is a criterion for the moment, the freezing time (period) is chosen so as to become the freezing size below it. iv. It is described how to decide the freezing time t.

When making it freeze to the size near the freezing limit size c, the time width is selected so as to almost reach the value c (that is, to reach the saturation state on the curve). When setting it as the size not close to the freezing limit size c, there is also a method to solve the numerical formula 4 alike Figure 3.

Utilizations of the numerical formula 3 and the numerical formula 4 are explained.

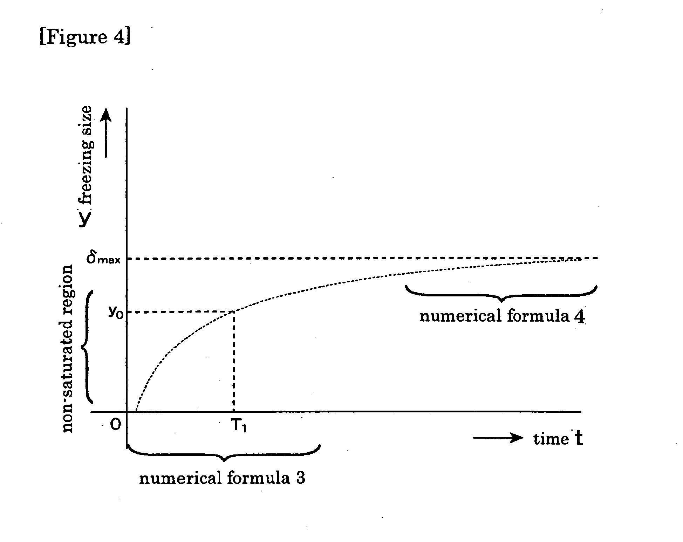

Since, in the numerical formula 3, the focus size is also the target freezing size $y_0$, values A, B and $y = y_0$ become fixed values, and the time t can be found by solving the numerical formula 3. On the other hand, when making it freeze to the size near the freezing limit size c, utility time to reach saturation is found by the numerical formula 4. This found time t is equivalent to the freezing time width $T_1$. Figure 4 shows this relation for simulation. $\delta_{max}$ is the maximum limit freezing size c, and is the value in the saturation state on the curve. The defrosting time $T_2$ is also calculated by the similar view. The experimental data in Figure 2, Figure 3, Figure 13 and Figure 14 change also with the kind of the freezing gas, the sending-out speed of the freezing gas and the diameter of the probe. The various values of A and B are asked based on the kind of freezing gas, its sending-out speed, the diameter of the probe and the organ oprtion, and stored in the memory of the computer 59; at the occasion of the treatment, the corresponding and applicable values of A and B are read out, the focus size y is inputted, and the time width t is asked by the numerical formula 3.

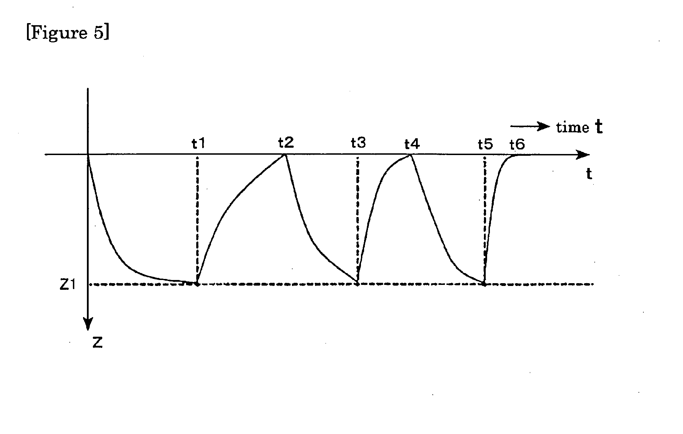

An example of freezing · defrosting sequences is shown in Figure 5. A trasvers shows the time t and a vertical axis shows the freezing size Z. $Z_1$ is the maximum freezing size (it is not the maximum limit in the example of use of the numerical formula 3, and it is near the maximum limit size in the example of use of the numerical formula 4). The sequence shown in this Figure is as follows;

$0 \sim t_1$ ... 1st freezing section
$t_1 \sim t_2$ ... 1st defrosting section
$t_2 \sim t_3$ ... 2nd freezing section
$t_3 \sim t_4$ ... 2nd defrosting section
$t_4 \sim t_5$ ... 3rd freezing section
$t_5 \sim t_6$ ... 3rd defrosting section.

0 - $t_2$ is the 1st cycle, $t_2 \sim t_4$ is the 2nd cycle and $t_5 \sim t_6$ is the 3rd cycle. In the Figure, the cycle widths was made as 1st cycle > 2nd cycle >3rd cycle. This is because that the effect of freezing is noticeable in 1st freezing-defrosting and the freezing effect can be continued with the energy less than that in 2nd and 3rd. Obviously, there is also an example having the same time width.

In the example using the numerical formula 3, the defrosting is in a reverse relation to the numerical formula 3, such as the numerical formula 5, for example.

[Numerical Formula 5] $$Z = C - D \ln(t)$$

Even in this expression, C and D are values obtained in advance as of A and B (usually C = B, D = A) and Z is the defrosing size (this is also the freezing size), and, by solving the numerical formula 5. time t, i.e., the aforementioned $T_2$ is obtained. Since values C and D change also with the inflow speed of the defrosting gas, C and D are settled based on these various parameters (the focus portion, the inflow speed, the kind of defrosting gas) and stored in the memory. They are read out at the time of the determination of the treatment sequence and the defrosting time width is decided according to the defrosting size.

A necrosis of a focus treatment portion depends on the freezing time width $T_1$ and the number n of the cycle, the freezing gas, and the inflow speed of the freezing gas. Especially, since the cycle number n is the frequency in which the freezing and the defrosting are repeated, there are many examples that the necrosis is difficult in only 1 time of the cycle and there are many examples that the complete necrosis is accomplished by using 2 cycles ~ 5 cycles. When the number of the cycle becomes large, the treatment time becomes long and hence the pains of the patient becomes great, and when the number of the cycle becomes small, it becomes difficult to accomplish the complete necrosis. The proper number n of the cycle is selected so as to mutually compensate the such shortages.

The number of the cycle is explained further, here.

The number n of the cycle is selected so as to accomplish the treatment effect without the patient's pains. The patient's pain is that the treatment time becomes long, and the treatment effect is that the focus treatment portion can be necrotized. In the values more than n = 1, n = 2 ~ 5 was the practical value. Although the treatment time became long compared with n = 1, the effect of necrosis was fully accomplished. This was confirmed by the experiments of Kansei Iwata, et al., the inventors of this application. The example of n = 2 is explained, hereinafter.

The freezing of the 1st time is a treatment for the focus treatment portion being the living body tissue (this is the living body tissues, regardless to tumor or normal cell, as a group of cells surrounding the air chamber in lung), and the freezing of the 2nd time is for the defrosting result of the 1st time. In the freezing of the 1st time for freezing the living body tissue, a frozen body (ice block) and a portion in semi-frozen state in the outside of its circumference appear. This portion of the semi-frozen state is a big enlarged area as compared with the body. The outside of the semi-frozen state portion is in a normal living body state.

When the defrosting is performed to this freezing, the frozen body liquefies and the semi-frozen state portion also requefies according to it. In such a liquefied portion, it is simultaneously accompanied by bleeding.

In the freezing of the 2nd time, the frozen body in a strong liquefaction freezes promptly and the semi-frozen state part in a weak liquefaction also freezes successively. That is, the freezing of 1st time was mainly for the freezing of the frozen body, in the 2nd freezing, enlarged semi-frozen state portion surrounding it is also frozen. Thus, the necrosis of the living body tissue is performed including the semi-frozen state portion. The defrosting is carried out after the completion of the freezing.

In the 2nd time, the semi-frozen state may be disregarded and the freezing fuction of the same coefficient as the 1st time may be used. However, in taking a semi-frozen state into consideration, the coefficiet is settled considering the freezing to the frozen body and also to the semi-frozen state. In this case, when n = 2 in treatment planning by the example using the numerical formula 3, it is settled the coefficients A, B, C and D according to each cycle, and it is preferrable to settle the focus treatment portion to the size of the semi-frozen state enlarged in the 1st cycle.

Of course, there is an example that the necrosis effect is also accomplished with the example of n = 1. Further, n = 3 ~ 5 is an example that the 3rd ~ 5th cycle accomplishes the further necrosis effect.

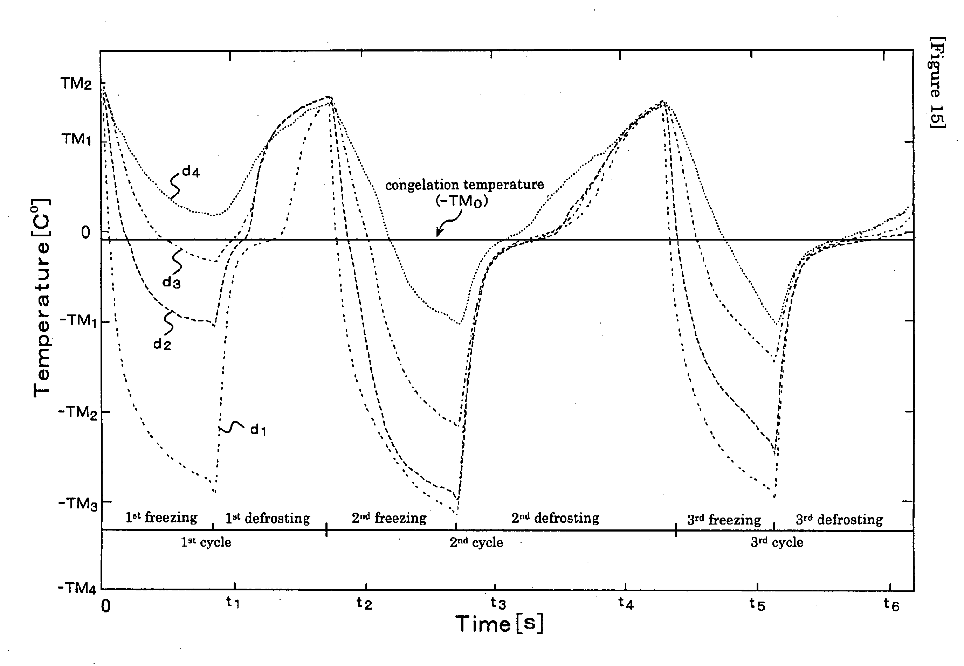

Figure 15 is an explanatory view of the frozen body and the semi-frozen state aroud it. This figure is the example figure for confirmation of the experiments, in the example of 3 cycles, of the temperature TM and the time t at a position d of each portion on a concentric circle from a freezing center. The position d of the each portion shows the diameter on the 4 concentric circles having the relation of $d_1 < d_2 < d_3 < d_4$, such as $d_1 = 4$ mm, $d_2 = 6$ mm, $d_3 = 8$ mm and $d_4 = 10$ mm, for example. In Figure 15, the temperature TM measured on each point of the 4 concentric circles in the process of 3 cycles is shown. Though the temperature is $TM_1 = 20°C$, $TM_2 = 40°C$, $-TM_1 = -20°C$, $-TM_2 = -40°C$, $-TM_3 = -60°C$ and $-TM_4 = -80°C$, the diameter and the temperature are mere one example.

The points recognized according to Figure 15 are enumerated.

EP 2 305 158 A1

(1) Temperature $-TM_0$ being a little lower than 0°C was regarded as the congelation temperature.

(2) The interval of the 2nd cycle is longer than that of the 1st cycle.

(3) Smaller diameter $d_1$ freezes promptly, and it reaches the temperature $-TM_2$ in the 1st cycle. Larger diameter $d_4$ freezes late. It does not freeze in the 1st cycle but freezes, for the first time, in the 2nd cycle. In the 3rd cycle, the freezing temperature further becomes low. The diameters $d_2$ and $d_3$ carry out the intermediate behaviour.

(4) Thus, it is understood from the Figure 15 that the nearer to the freezing center the freezing progresses promptly, the farther it takes a long time for freezing. The portion frozen in the 1st cycle is the aforementioned freezing body, the no-frozen portion existing in its circumference becomes in the semi frozen state. The frozen mass in the 2nd cycle is larger than that of the 1st cycle, and it sometimes becomes more than double.

(5) In addition, the treatment portion is fundamentally maintained in a temperature of, usually, the patient's body temperature (36 °C etc. variously). The normal temperature differs variously according to the region of the body, such as relatively high in the region near the artery Therefore, the freezing and the defrosting conditions change according to these regions and, so, each coefficient of the freezing function and the defrosting function also takes various values.

Operation of an embodiment of Figure 1 is explained.

(1) Generation of treatment planning data including treatment sequence (freezing defrosting sequence).

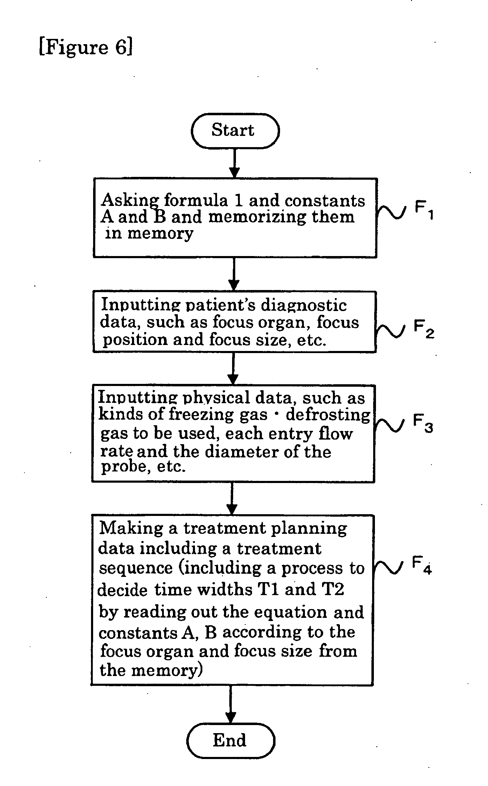

The generation flow of the treatment planning data utilizing the numerical formula 3 is shown in Figure 6. In the flow $F_1$, the equation of the numerical formula 3 and the constants A, B (C and D are also included) are asked accoding to the organs, such as lung and liver, the diameter of the probe and a kind of gas, etc., and stored in the memory of the computer 59. In the flow $F_2$, patient's diagnostic data (a focus organ, a focus position, a focus size and a classification of focus, etc.) is inputted.

In the flow $F_3$, the physical data of each mechanical system for a treatment, such as kinds of the freezing gas and the defrosting gas to be used, each entry flow rate and the diameter of the probe, etc. is inputted. In the flow $F_4$, the treatment planning data including the treatment sequence is made by using the data of the flows $F_2$ and $F_3$ and reading out A, B and the equation etc. from the memory. This treatment sequence is, in a narrow sence, freezing defrosting sequence wherein the freezing · defrosting is made as 1 cycle, and comprises the number n of cycle, the freezing time width $T_1$ and the defrosting time width $T_2$. $T_1$ and $T_2$ are obtained by the numerical formula 3 and the numerical formula 5. The number n is settled by an experimental value or according to $T_1$ and $T_2$. It is, in a broad sense, includes the treatment process before and after the freezing defrosting sequence. For example, it includes each work of the initialization and the attachment, etc. of various kinds of monitoring apparatus (a display, an electrocardiograph, an X-ray apparatus, etc.) before entering into the freezing · defrosting sequence.

As other treatment planning data, it is included that the treatment procedure data from the start to the end of the treatment, the data of notes in the treatment (for example, the other organ exists in near), and the operating procedure data of the gas supply system (51 - 56) and the exhaust system (57) for the freezing defrosting which is a part thereof.

(2) Execution of the treatment

The treatment is performed based on said generated treatment planning data. Although the flow of the whole treatment is omitted, the one concerning to this invention is execution of the freezing defrosting sequence.

According to the shift to the freezing defrosting sequence from the computer 59, the freezing treatment is carried out by the inflow of the freezing gas, in case of the freezing, through 51→53→55→56→60 (more than 1 or 2 among them) by means of the control portion 58. In case of the defrosting, by the inflow of the defrosting gas through 52→54→55→56-->60 (more than 1 or 2) by means of the control portion 58, the defrosting is carried out.

Various methods for execution of the treatment are shown.

(1) There is a method of mostly full automation by attaching the manipulator to the probe.

(2) The manipulator is attached to the probe, and a person for the operarion operates the probe through the manipulator and controls the inflow and the outflow of gas according to the procedure displayed on a screen corresponding to the treatment sequence. In this method, the treatment sequence only displays the operation data which needs for the treatment operation on the screen, and hence the person for the operation becomes to treat according to the operation data.

(3) There is also a method which is interim between abovementioned (1) and (2), i.e., the method wherein a part is automated and a part is operated on manual.

(4) The example by the numerical formula 4 is explained.

In the example by the numerical formula 4, when the treatment portion is settled and the treatment size is settled, the probe having the freezing limit size corresponding to the treatment size is selected. Of course, it is also premised to settle the kind of gas to be used. The freezing cycle is settled in consideration of the treatment effct. Others are the same as that of the numerical formula 3.

Other embodiment is explained.

In the cryotherapy device wherein the freezing is performed by making the liquefied gas, for example, liquid nitrogen gas (for example -195°C flow in, the liquefied gas for freezing may remain in the probe at the time of defrosting. Since there is a possibility of evaporating at a stretch and exploding when the defrosting gas (including liquid) is flowed in this state, it is difficult to realize the cryotherapy device wherein the liquefied gas is flowed in. Then, the embodiment which solves such a problem is shown below. This embodiment is that the purge period for purging the liquefied gas is employed in the freezing period, and the vaporized gas of the liquefied gas concerned is utilized for the purge.

Hereafter, the embodimet of this viewpoint is explained.

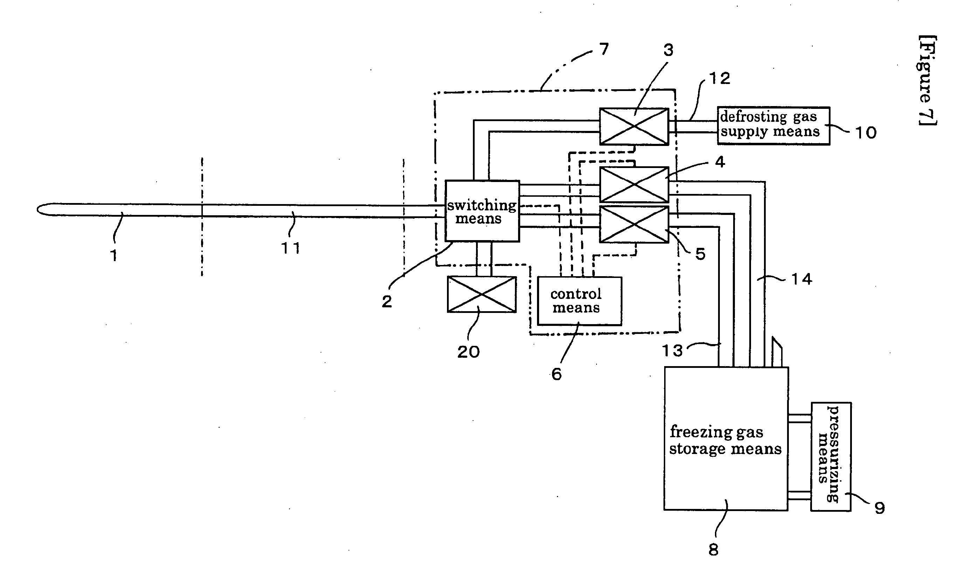

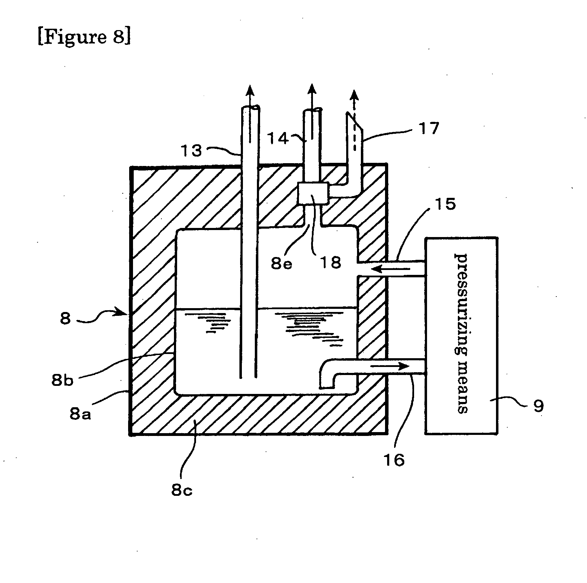

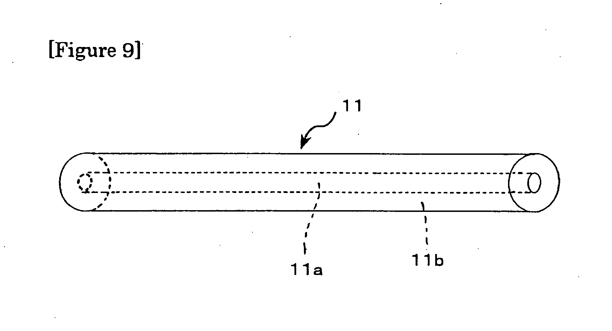

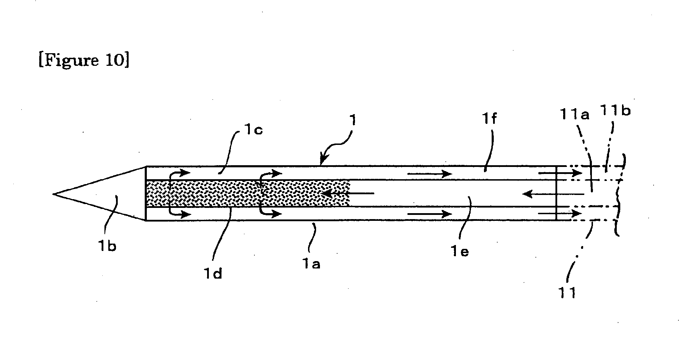

Figure 7 is a structural sample view of the mechanical system of the cryotherapy device, i.e., the gas supply-exhaust system 100 and the probe system 300 (example of 1 probe), Figure 8 is an enlarged sectional view of a liquefied gas storage means, Figure 9 is a perspective view of the gas supply-exhaust pipe connecting the cryotherapy device and the probe, and Figure 10 is an enlarged sectional view of the probe.

The cryotherapy device shown in Figure 7 comprises the probe 1 composing of the freezing treatment apparatus, the gas switching control means 7 composing of the gas switching means 2 which supply freezing gas and defrosting gas altanatively to the probe 1 in the treatment and control means 6 which controls the gas switching means 2 and plural opening-shutting valves 3, 4 and 5, the freezing gas storage means 8 used as the freezing gas supply source and connected to the gas switching control means 7, the pressurizing means 9 which pressurizes the inside of the freezing gas storage means 8 at a predetermined pressure, and the defrosting gas supply means 10 connected to the 1st switching valve 3 of the gas switching control means 7, and the gas switching control means 7 is connected to the probe 1 by the gas supply-exhaust pipe 11 which has a flexibility.

This cryotherapy device is a mechanical system, and, although not illustrated, it is controlled by the control section 58 of the computer etc. shown in Figure 1.

The control is realized by control of the interactive man-machine method through the screen according to the directions of the operator (the person for the operation). The main points of the control are the freezing · defrosting sequences being the treatment processing and are explained later.

The probe 1 composing of the freezing treatment apparatus consists of, as shown in Figure 8, the probe body 1a and the stabbing portion 1b which forms the tip portion of the probe 1. The probe body 1a is formed by the stainless steel pipe of 2 mm - 3 mm in the outer diameter and the evaporation chamber 1c is located in the tip side of the probe body 1a. The evaporation means 1d for evaporating the liquefied freezing gas is formed in the central portion of the evaporation chamber lc.

The evaporation means 1d comprises the perforated pipe having a plurality of small through-holes, from which the liquefized freezing gas spouts into the chamber 1c, in the peripheral wall of the thin stainless steel pipe of about 0.6 mm in the outer diameter, for example, and is set the central portion of the chamber 1c so as to be parallel to the axis of the probe body la. Its one end side reaches the stabbing portion 1b of the probe body 1a and the other end side is connected to the one end side of the gas outward path 1e provided on the base end side of the probe body la.

The gas outward path 1e consists of, as same as the evaparation means 1d, the thin stainless steel pipe of about 0.6 mm in the outer diameter, and is set the central portion of the probe body 1a so as to be parallel to the axis and to provide the freezing gas and the defrosting gas to the perforated pipe ld. The gas return path 1f for exhausting the gas spouted from the evaporation means 1d to the evaporation chamber 1c is formed between the outer surface of the gas outward path 1e and the inner surface of the probe body la.

The other end sides of the gas outward path 1e and the gas return path 1f are connected respectively to the one end sides of the gas outward path 11a and the gas return path 11b of the gas supply-exhaust pipe 11 connected to the base end side of the probe body la.

The gas supply-exhaust pipe 11 has the double pipe structure, as shown in Figure 9, of the inner pipe 11c and the outer pipe 11d consisting of an elastic flexible pipe, so that it may not become the hindrance of the operation to stab the probe 1 to the affected portion.

The inside of the inner pipe 11c is the gas outward path 11a and the path between the inner pipe 11c and the outer pipe 11d is the gas return path 1b. The other end side of the gas supply-exhaust pipe 6 is connected to the gas switching means 2 provided to the gas switching control means 7.

The gas switching control means 7 comprises, as shwn in Figure 7, the 1st, 2nd and 3rd opening-shutting valves 3, 4 and 5 consisting of a plurality of electromagnetic valves, the gas switching means 2 consisting of the multi-way valve for supplying gas, which is supplied selectively by the 1st, 2nd and 3rd opening-shutting valves 3, 4 and 5, to the probe body 1a through the gas supply-exhaust pipe 11, and the control means 6 for controlling the switching of the 1st, 2nd and 3rd opening-shutting valves 3, 4 and 5 and the gas switching means 2.

Opening and shutting control of the 1st, 2nd and 3rd opening-shutting valves 3, 4 and 5 and the gas switching means 2 are carried out by the switching operation timing beforehand programmed in the control means 6, and, to the 1st switching valve 3 of the gas switching control means 7, the defrosting gas supply means 10 to supply the desfrosting gas, such as the hellium gas, is connected through the defrosting gas supply pipe 12.

On the other hand, to the 2nd switching valve 4 and the 3rd switching valve 5, the freezing gas storage means 8 is connected.

The freezing gas storage means 8 accommodates, as shown in Figure 8, the storage tank 8b of airtight structure wherein the freezing gas (for example $CO_2$ liquefied gas) is stored in a box-like case 8a. The thermal insulator 8c is filled between the case 8a and the storage tank 8b, and the inside of the storage tank 8b is always maintained at a predetermined temperature.

In the storage tank 8b, there is the liquefied freezing gas of about 1/2 of full capacity in the lowere part thereof, and the vaporized freezing gas of about 1/2 of full capacity in the upper part thereof.

The gas of the freezing gas stored in the upper part of the storage tank 8b has the temperature of mostly same as that of the liquefied freezing gas. This freezing gas (hereinafter called a purge gas) is utilized as a purge gas for discharging the freezing gas and the defrosting gas stagnated in the probe body la.

The freezing gas feed pipe 13 and the purge gas feed pipe 14 are set in the upper part of the storage tank 8b.

The lower end of the freezing gas feed pipe 13 reaches near the bottom of the storage tank 8b and is immersed in the portion of the liquefaction of the freezing gas so as to supply only the liquefied defrosting gas. The lower end of the purge gas feed pipe 14 is connected to the opening 8e on the upper surface of the storage tank 8b so that only the purge gas can be supplied.

The pressurizing means 9 is connected to the storage tank 8b, and the inside of the storage tank 8b is always pressurized in the predetermined pressure.

The pressurizing means 9 consists of, for example, a pump, and is connected to the lower portion of the storage tank 8b via the suction pipe 16 to inhale the freezing gas stored in the lower portion of the storage tank 8b. The freezing gas pressurized at the predetermined pressure by the pressurizing means 9 is exhaled to the purge gas stored in the upper portion of the storage tank 8b via the discharge pipe 15.

It is set in the purge gas feed pipe 14 that the safety valve 18 for preventing the inside of the storage tank 8b from becoming higher than the upper limit pressure by the discharge of the purge gas in the storage tank 8b to the air via the exhaust pipe 17 when the pressure in the storage tank 8b reaches the upper limit pressure settled beforehand.

The numeral 20 in Figure 7 is the exhaust means consisting of the exhaust valve to discharge the freezing gas, the defrosting gas and the purge gas to the air, and is connected to the gas switching means 2.

Now, the concrete method in the case of the treatment of a malignant tumor is explained utilizing the cryotherapy devices of the embodiments shown in Figure 7 ~ Figure 10.

First, the probe 1 is inserted into the inside of the patient's body so that the tip of the probe 1 of the freezing treatment apparatus arrives at the malignant tumor tissue, and the tip portion of the probe 1 is made to penetrate to the patient's affected portion.

Next, the gas switching control means 7 is switched to the freezing treatment mode, and the freezing treatment is started. After the 1st switching valve 3 is closed, the 2nd switching valve 4 is opened and the 3rd switching valve 5 is closed according to the operation timing beforehand programmed in the control means 6, the gas switching means 2 is switched in the direction so that the 2nd switching valve 4 and the probe 1 becomes in the open state each other. Hence, the purge gas stored in the state of pressurized to, for example, 150kg/cm2 at the maximum by the pressurizing means 9, in the upper portion of the storage tank 8b is supplied to the probe 1 through the gas outward path 11a of the gas supply-exhaust pipe 11 and the purge process is carried out.

The purge gas supplied to the probe 1 reaches the perforated pipe 1d through the gas outward path 1e in the probe body 1a, is exhausted into the evaporation chamber 1c through the small through-holes provided in the peripheral wall of the perforated pipe 1d, reaches the gas switching means 2 through the gas return path If in the probe body 1a and the gas return path 11b of the gas supply-exhaust pipe 11, and is exhausted to the air from the exhaust means 20 connected to the gas switching means 2.

Thus, the air remained in the gas supply-exhaust pipe 11 and the probe 1 is purged, and the purge process of the air is completed.

Next, after the 1st and 2nd switching valves 3 and 4 are closed, and the 3rd switching valve 5 is opened by the control means 6, the gas switching means 2 is switched in the direction so that the 3rd switching valve 5 and the probe 1 becomes in the open state each other. Hence, the liquefied freezing gas stored in the state of pressurized to, for example, 150kg/cm2 at the maximum by the pressurizing means 9, in the lower portion of the storage tank 8b is supplied to the probe 1 through the gas outward path 11a of the gas supply-exhaust pipe 11 and the freezing process is carried out.

The freezing gas supplied to the probe 1 reaches the evaporation means 1d consisting of the perforated pipe through the gas outward path 1e in the probe body 1a, and is spouted in misty state into the evaporation chamber 1c through the small through-holes provided in the peripheral wall of the perforated pipe ld. Since it is evaporated in the evaporation chamber 1c, the surrounding heat is taken away by the evaporation heat, the probe 1d is cooled, and hence the freezing of the affected portion is started.

The freezing gas evaporated in the evaporation chamber 1c reaches the gas switching means 2 through the gas return path 11b of the gas supply-exhaust pipe 11, and is exhausted through the gas switching means 2 to the air from the exhaust means 20.

After the freezing process is completed by the progress of the time programmed beforehand, the freezing process is swiched to the defrosting process. In the transition period from the freezing process to the defrosting period, the purge process of freezing gas is carried out.

That is, after the freezing process of the affected portion is completed, the control means 6 makes the 1st switching valve 3 in close, 2nd switching valves 4 in open and the 3rd switching valve 5 in close, and switches the gas switching means 2 in the direction so that the 2nd switching valve 4 and the probe 1 becomes in the open state each other.

Thus the purge gas stored in the upper part of the storage tank 8b is supplied to the probe 1 through the gas outward path 11a of the gas supply-exhaust pipe 11, and the purge gas supplied to the probe 1 reaches the evaporation means 1d through the gas outward path 1e, and is exhausted into the evaporation chamber 1c through the small through-holes provided in the peripheral wall of the evaporation means ld.

Further, since it reaches the gas switching means 2 through the gas return path If in the probe body 1a and the gas return path 11b of the gas supply-exhaust pipe 11 and is eshausted to the air through the exhaust means 20 from the gas switching means 2, the remained gas, which is not evaporated, in the perforated pipe 1d of the probe 1 and the liquefied freezing gas remained in the evaporation chamber 1c are exhausted with the purge gas to the air through the exhaust means 20, and hence all the freezing gas remained in the probe is exhausted.

Since the freezing gas vaporized in the storage tank 8b is used for the purge gas for dischege the liquefied freezing gas remained in the probe 1, the purge gas has almost same temperature as that of the liquefied freezing gas and thus the freezing gas remained in the probe 1 is discharged without changing the temperature in the probe 1.

After the purge process is completed by the discharge of the remained freezing gas in the probe 1, it is changed to the defrosting process. The 1st switching valve 3 is changed to open, the 2nd and 3rd switching valves 4 and 5 are changed to close and then the gas switching means 2 is switched in the direction so that the 1st switching valve 3 and the probe 1 becomes in the open state each other by the control means 6. The defrosting gas such as hellium is supplied to the probe 1 through the gas outward path 11a of the gas supply-exhaust pipe 11 from the defrosting gas supply means 10 connected to the 1st switching valve 3, and hence the defrosting process is carried out.

The defrosting gas supplied to the probe 1 reaches the perforated pipe 1d through the gas outward path 1e in the probe body 1a, is vaporized in the evaporation chamber 1c by the spout in misty state into the evaporation chamber 1c through the small through-holes provided in the peripheral wall of the perforated pipe 1d, and the defrosting of the affected portion frozen by the freezing gas is started.

The defrosting gas evaporated in the evaporation chamber 1c reaches the gas switching means 2 through the gas return path If in the probe body 1a and the gas return path 11b in the gas supply-exhaust pipe 11, and is exhausted to the air from the gas switching means 2 through the exhaust means 20.

The input amount of heat added to the affected portion by the freezing gas is calculated by the theoretical formula, and it is required, for defrosting the frozen affected portion by the defrosting gas, to apply the amount of heat equivalent to that of the freezing, by the defrosting gas, to the affected portion. It is omitted here as to the theoretical formula.

After the defrosting process programmed aforehand is completed, the defrosing process is changed to the purge process, and then the purge process is carried out again.

By the freezing process and the defrosting process are alternately repeated by repeating supply of the freezing gas, the purge gas and the defrosting gas alternately, the malignant tumor tissue of the affected portion is necrotized and the treatment effect by the cryotherapy comes to be acquired.

Since the freezing and the defrosting of the affected portion is carried out, in a short time, efficiently by putting the purge process between the freezing process and the defrosting process, the treatment time becomes short and hence the patient's pain is reduced.

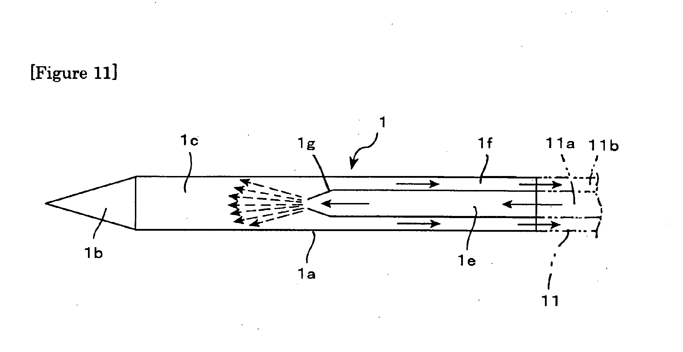

Though, in the above-mentioned embodiments, the evaporation means 1d consisting of the perforated pipe is provided in the anterior portion of the probe body 1a, the liquefied freezing gas is vaporized by spouting in misty state into the evaporation chamber 1c through the small through-holes provided in the peripheral wall of the perforated pipe, and the affected portion is freezed by the evaporation heat generated at this time, it may be made, as shown in Figure 11, that the evaporation means 1g consisting of the nozzle is provided in the anterior portion of the probe body 1a, the liquefied freezing gas is vaporized by spouting in misty state into the evaporation chamber 1c from this evaporation means 1g, and the affected portion is freezed by the evaporation heat generated at this time.

Although the unnessesary freezing gas, purge gas and defrosting gas were discharged from the exhaust means 20 to the air, it may be made to return the freezing gas and the purge gas to the storage tank 8b, and the defrosting gas to the defrosting gas supply means 10.

Although the probe 1 stabbed directly the patient's affected portion in the freezing treatment, it may be made that an introducer beforehand stabbs the affected portion and then the probe 1 stabbs the patient's affected portion using the introducer as the guide. Also it may be made that a outer sheath pipe stabbs the patient's affected portion using the introducer as the guide; the introducer is drawn out from the outer sheath pipe when the tip of the outer sheath pipe penetrates the affected portion; in this state, the probe 1 is inserted into the outer sheath pipe and stabbs the affected portion;and after the outer sheath pipe is extracted, the freezing and the defrosting of the affected portion is carried out by the probe 1.

Although the example of the cryotherapy method for the malignant tumor utilizing the probe was explained, it can be applied to overall cryotherapy devices which are used for the deseases that the cryotherapy method is effective.

The purge period is a short time compared with the freezing time width and the defrosting time width, and hence it is rare to affect the freezing and the defrosting. However, in case that the purge period is taken in consideration, it should be considered that, in the purge period of the freezing gas, for example, how it gives the freezing the influence, and that, in the purge period of the defrosting gas, how it gives the defrosting the influence. Its degree of incidence can be settled variously to the values defined experientially, and to the values settled in consideration of the freezing energy and the defrosting energy, etc.

There is the following way, for example.

In explaining the cryotherapy method which treats the affected portion by utilizing said constituted cryotherapy device, the treatment sequence is explained first.

The sequence of the treatment process is the processes repeating plural cycles (such as 2 times or 5 times), when the freezing process time $T_1$ and the defrosting process time $T_2$ is made as 1 cycle. There are both cases, $T_1 = T_2$ and $T_1$ $T_2$. $T_1$ and $T_2$ may change for every cycle. For example, times in 2nd and 3rd cycles are made shorter than $T_1$ and $T_2$ in the 1st cycle. This is because that the freezing and the defrosting are performed in the 1st cycle and hence lesser energy of the freezing and the corresponding lesser energy of the defrosting are sufficient after the 2nd cycle.

The freezing process time $T_1$ is the time width for freezing. Specifically, it is the total value of the period for freezing actually (actual freezing period) $T_{11}$ by opening the 3rd switching valve 5 and hence carrying out the freezing gas to the tip in the probe 1, and the purge period $T_{12}$ for purging compulsorily the freezing gas to the outside from the inside of the probe 1.

The defrosting process time $T_2$, which starts at the end of the purge, is the total value of the period for defrosting actually (actual defrosting period) $T_{21}$ by opening the Ist switching valve 3 and hence performing the defrosting gas to the tip of the probe 1, and the purge period $T_{22}$ for purging the defrosting gas compulsorily to the outside from the inside of the probe 1.

Although the freezing in the freezing process time $T_1$ is basically settled by the real freezing period $T_{11}$, the freezing continues by the influence of the freezing gas remained during the purge, since it is difficult to purge the freezing gas immediately even in the purge period $T_{12}$.

Therefore, in order to realize the freezing effect, it is necessary to consider the Tn and $T_{12}$ in series. Then the above-mentioned numerical formula 3 is used.

On the other hand, the freezing gas is decreased gradually by the purge during the purge period and hence the freezing ability becomes small. Therefore, it only has to consider the degree of influence to the freezing size in the purge period in consideration of the decrease of the freezing ability. There is a view as follows.

(1) There is a way that the purge period $T_{12}$ which is fixed by the purge speed and the total amount of the freezing gas is settled; the freezing size yo which grows in the period $T_{12}$ is obtained experimentally or theoretically; and these are added to the numerical formula 2. That is,

$$[\text{Numerical Formula 6}] \qquad y = A \ln(t) - B + y_0.$$

In this formula, t is obtained by the replacement of y with the focus size So for the treatment. This t obtained is the period $T_{11}$.

(2) There is a way that the quantity of the freezing gas remained in the probe is set to C; the quantity of the purge gas carried out in a unit time is set to D; and these are added to the numerical formula 6. That is, when y = So, the time width t is obtained following formula

$$[\text{Numerical Formula 7}] \qquad y = A \ln(t) - B + (C/D) \cdot t.$$

This time width t is ($T_{11} + T_{12}$). The allotment of T11 and T12 is decided by the proportional distribution of {A In (t) - B} and (C/D) . t.

(3) In the actual treatment, there is also an example which is not y = So. In this case, it is solved by that the relation between y and So is asked in advance and So under that relation is replaced by y. It is also the way to solve by that the So is settled larger than the freezing size y, such as So = k y (now, k > 0). Further, there is the example that So is settled so as to include the doubtful portion near the circumference of the focus by settling So larger than the actual focus size.

(4) It is also the same in the examples of the numerical formula 4.

Although the numerical formula 3 and the numerical formula 5 are the approximation of the natural logarithmic function and the numerical formula 4 is the approximation of the exponential function, it does not adhere to the natural logarithmic function when it has, as a result of the statistical regression analysis, a higher degree of regression analysis than the natural logarithmic function. There is also function expression with high order of approximation by the fixed time variable, and this is not barred, either.

**Brief Description of the Drawings**

[Figure 1] It is the figure of whole embodiment of the cryotherapy device of this invention.
[Figure 2] It is the example of the animal experimental data.
[Figure 3] It is the example of the animal experimental data.
[Figure 4] It is the figure of the example of the function expression of the animal experimental data.
[Figure 5] It is the figure of the example of the freezing defrosting sequence of this invention.
[Figure 6] It is the figure of the process flow chart of this invention.
[Figure 7] It is the general structural figure of the mechanical system of the cryotherapy device provided with the freezing medical apparatus which is the embodiment of this invntion.
[Figure 8] It is the enlarged sectional view of the freezing gas storage means which composes the cryotherapy device of the embodiment of this invention.
[Figure 9] It is the enlarged perspective view of the gas supply-exhaust pipe connecting the freezing treatment apparatus and the cryotherapy device which is the embodiment of this invention.
[Figure 10] It is the enlarged sectional view of the freezing treatment apparatus of the embodiment of this invention.
[Figure 11] It is the enlarged sectional view of the modifed example of the freezing treatment apparatus which is the embodiment of this invention.
[Figure 12] It is the figure of the exhaust system of th embodiment of this invention.
[Figure 13] It is the example of the animal experimental data.
[Figure 14] It is the example of the animal experimental data.
[Figure 15] It is the figure of the example of temperature change, of this invention, by the freezing and the defrosting in the time passage on a plurality of circumferences of the treatment portion.

**Explanation of Notations**

1        Probe
1a       Probe body
1c       Evaporation chamber
1d       Evaporation means
2        Gas switching means
6        Control means
8        Freezing gas storage means
9        Pressurizing means
10       Defrosting gas supply means
11       Gas supply-exhasut pipe
100      Gas supply-exhaust system
200      Control system
300      Probe system

**Claims**

1. A cryotherapy planning device comprising, in a cryotherapy device wherein a freezing and a defrosting can be performed in a freezing period which freezes a treatment portion and in a defrosting period in which its defrosting is performed, by a freezing probe having a predetermined section size, that said freezing period and said defrosting period are settled according to an organization and a focus treatment size of the treatment portion.

2. A cryotherapy planning device comprising, in a cryotherapy device wherein a freezing and a defrosting can be performed in a freezing period which freezes a treatment portion and in a defrosting period in which its defrosting is performed, that said freezing period and said defrosting period are settled according to a section size of a freezing probe, and an organization and a focus treatment size of the treatment portion.

3. A cryotherapy planning device wherein a relation between a kind of diameter of a freezing probe and a focus treatment size, obtained by a diameter size of the freezing probe and a freezing limit size settled according to the diameter size, is memorized as data, and, by using this data, a freezing probe to be used and a focus treatment size are determined.

4. A cryotherapy planning device wherein a relation between a diameter size of a freezing probe, a freezing limit size settled according to the diameter size and a time width approaching to the freezing limit size, is memorized as data, and, by using this data, a freezing probe to be used, a focus treatment size and a freezing time are determined.

5. A cryotherapy planning device wherein a relation between a diameter size of a freezing probe, a freezing limit size settled according to the diameter size and a time width approaching to the freezing limit size, is memorized as data, and, by using this data, a freezing probe to be used, a focus treatment size, a freezing time, a defrosting period and/or a repetition of cycle of its freezing and defrosting are determined.

6. A cryotherapy planning device according to Claim 5, wherein said relation is controlled by a following equation settled by a diameter of a freezing probe, when y is a freezing size and c is a freezing limit size,
   y = a exp(bt) + c
   wherein exp is an exponential function, a, b and c are coefficients settled by a treatment portion and a diameter of a probe, being in a relation of a < 0, b < 0, c > 0, and c is the freezing limit size.

7. A cryotherapy device comprising a freezing probe; a gas control means to send a freezing gas for freezing a treatment portion during a freezing period and to send a defrosting gas for its defrosting during a defrosting period, to the freezing probe; means for setting a freezing probe to be used, a focus treatment size, a freezing time T1 and a defrosting time T2 by utilizing memorized data of a relation between the diameter size of the freezing probe, a freezing limit size settled according to the diameter size and a time width approaching to the freezing limit size; and commanding means for sending a control command to said gas control means so as to freeze for the settled freezing time T1 and to defrost for defrosting time T2.

8. A cryotherapy device according to Claim 7, wherein said relation is controlled by a following equation settled by a diameter of a freezing probe, when y is a freezing size and c is a freezing limit size,
   y = a exp(bt) + c
   wherein exp is an exponential function, a, b and c are coefficients settled by a treatment portion and a diameter of a probe, being in a relation of a < 0, b < 0, c > 0, and c is the freezing limit size.

9. A freezing treatment period planning device which obtains a freezing period $T_1$ for freezing a treatment portion and a defrosting period $T_2$ to defrost it by utilizing a freezing probe, wherein the freezing period $T_1$ for freezing the treatment portion surrounding the freezing probe by a freezing gas is settled based on a time t obtained by solving an equation of

$$y = A \ln(t) + B \text{ (ln is natural logarithm)}$$

wherein A and B are constants settled by an organization of the treatment portion and y is a focus treatment size and the defrosting period $T_2$ is settled based on a time t obtained by solving an equation of

$$Z = C - D \ln(t)$$

wherein Z is a defrosting size, and C and D are constants settled by an organization of the treatment portion.

10. A freezing treatment period planning device according to Claims 1 or 2, wherein, when a freezing period and a defrosting period are made as 1 cycle, the number of a repetition of the cycle is settled.

11. A freezing treatment period planning device according to one of Claims 3 ∼ 10, wherein a purge period for purging the freezing gas is added to the freezing period.

12. A cryotherapy device comprising a freezing probe, a gas control means to send a freezing gas for freezing a treatment portion during a freezing period and to send a defrosting gas for its defrosting during a defrosting period, to the freezing probe, means for setting a freezing period $T_1$ which is settled based on a time t obtained by solving an equation of

$$y = A \ln(t) + B \ (\text{ln is natural logarithm})$$

wherein A and B are constants settled by an organization of the treatment portion and y is a focus treatment size, and a defrosting period $T_2$ which is settled based on a time t obtained by solving an equation of

$$Z = C - D \ln(t) \ (\text{ln is natural logarithm})$$

wherein Z is a defrosting size, and C and D are constants settled by an organization of the treatment portion, and commanding means for sending a control command to said gas control means so as to freeze for the settled freezing period $T_1$ and to defrost for the defrosting period $T_2$.

13. A cryotherapy device according to one of Claims 7, 8 and 12, wherein said setting means settle the number of a repetition of cycle, when a freezing period and a defrosting period are made as 1 cycle, and said commanding means send control commands for freezing and defrosting to said gas control means according to the cycle.

14. A cryotherapy device according to one of Claims 7, 8 and 12, wherein said setting means add a purge period for purging a freezing gas in case that a liquid freezing gas is utilized to said freezing period, and said commanding means send control commands for freezing, defrosting and purging according to these periods.

15. A cryotherapy device according to Claim 14, wherein a gas of a vaporized gas generated in a liquid freezing gas source is used as the purge gas in the purge period.

16. A cryotherapy device according to one of Claims 7, 8 and 12 ∼ 15, wherein said gas control means has a negative pressure system for exhausting gas.

17. A cryotherapy device according to Claim 15, wherein said negative pressure mechanism has a negative monitoring sensor and said gas control means is stopped when said sensor detects a positive pressure turned from a negative pressure.

[Figure 1]

100 (gas supply-exhaust system)

53

55

56

60

#1

51

#2

52

54

58

#n

59

300
(freezing probe system)

57

200 (contol system)

EP 2 305 158 A1

[Figure 2]

[Figure 3]

[Figure 4]

[Figure 5]

[Figure 6]

```
                   ┌──────────────┐
                   │    Start     │
                   └──────────────┘
                          │
                          ▼
        ┌───────────────────────────────┐
        │ Asking formula 1 and constants│      F₁
        │ A and B and memorizing them   │  ∿
        │ in memory                     │
        └───────────────────────────────┘
                          │
                          ▼
        ┌───────────────────────────────┐
        │ Inputting patient's diagnostic│
        │ data, such as focus organ,    │  ∿   F₂
        │ focus position and focus      │
        │ size, etc.                    │
        └───────────────────────────────┘
                          │
                          ▼
        ┌───────────────────────────────┐
        │ Inputting physical data, such │
        │ as kinds of freezing gas ·    │
        │ defrosting gas to be used,    │  ∿   F₃
        │ each entry flow rate and the  │
        │ diameter of the probe, etc.   │
        └───────────────────────────────┘
                          │
                          ▼
        ┌───────────────────────────────┐
        │ Making a treatment planning   │
        │ data including a treatment    │
        │ sequence (including a process │  ∿   F₄
        │ to decide time widths T1 and  │
        │ T2 by reading out the equation│
        │ and constants A, B according  │
        │ to the focus organ and focus  │
        │ size from the memory)         │
        └───────────────────────────────┘
                          │
                          ▼
                   ┌──────────────┐
                   │     End      │
                   └──────────────┘
```

22

[Figure 7]

[Figure 8]

[Figure 9]

[Figure 10]

[Figure 11]

[Figure 12]

72

from probe — | exhaust surge tank | — | negative pressure sensor | to the computer 59 through the control sensor system

70

| negative pressure generating mechanism | — exhasust

71

[Figure 13]

[Figure 14]

[Figure 15]

**EP 2 305 158 A1**

## INTERNATIONAL SEARCH REPORT

| | International application No. |
|---|---|
| | PCT/JP2009/062629 |

A. CLASSIFICATION OF SUBJECT MATTER
A61B18/02(2006.01)i, A61F7/10(2006.01)i

According to International Patent Classification (IPC) or to both national classification and IPC

B. FIELDS SEARCHED

Minimum documentation searched (classification system followed by classification symbols)
A61B18/02, A61F7/10

Documentation searched other than minimum documentation to the extent that such documents are included in the fields searched
Jitsuyo Shinan Koho        1922–1996   Jitsuyo Shinan Toroku Koho   1996–2009
Kokai Jitsuyo Shinan Koho  1971–2009   Toroku Jitsuyo Shinan Koho   1994–2009

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2007-167100 A  (DGS Computer Co., Ltd), 05 July, 2007 (05.07.07), Full text; all drawings (Family: none) | 1-17 |
| A | JP 2004-41428 A  (GE Medical Systems Global Technology Co., L.L.C.), 12 February, 2004 (12.02.04), Full text; all drawings (Family: none) | 1-17 |

☒  Further documents are listed in the continuation of Box C.        ☐  See patent family annex.

| | | |
|---|---|---|
| * | Special categories of cited documents: | |
| "A" | document defining the general state of the art which is not considered   to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 27 August, 2009 (27.08.09) | 15 September, 2009 (15.09.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

| | INTERNATIONAL SEARCH REPORT | International application No. |
|---|---|---|
| | | PCT/JP2009/062629 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2006-516204 A  (Endocare, Inc.),<br>29 June, 2006 (29.06.06),<br>Par. No. [0045]<br>& US 2003/0078490 A1    & US 2004/0143181 A1<br>& US 2002/0016540 A1    & US 6485422 B1<br>& US 6139544 A          & US 2008/0154253 A1<br>& US 2002/0198518 A1    & US 6694170 B1<br>& US 6139544 A          & EP 1599134 A<br>& EP 1158922 A          & WO 2004/051409 A2<br>& WO 2000/072773 A1     & CA 2507289 A | 1-17 |

Form PCT/ISA/210 (continuation of second sheet) (April 2007)

## INTERNATIONAL SEARCH REPORT

International application No.

PCT/JP2009/062629

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:
   The inventions of claims 1, 2, 7-17 involve a special technical feature that a freezing time and an unfreezing time are determined depending on the tissue of the therapy region and a lesion side.
   The inventions of claims 3-6 involve a special technical feature that a freezing probe and a lesion therapy size. Therefore there is no special technical feature common to the two groups of the claims, and consequently the international application contains two inventions.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable,
the                             payment of a protest fee.

                          ☐ The additional search fees were accompanied by the applicant's protest but the applicable protest
                             fee was not paid within the time limit specified in the invitation.

                          ☐ No protest accompanied the payment of additional search fees.

Form PCT/ISA/210 (continuation of first sheet (2)) (April 2007)

**EP 2 305 158 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007167100 A
- JP 2007167101 A
- JP 2007295953 A